# EUROPEAN PATENT APPLICATION

(11) **EP 2 374 810 A1**
(43) Date of publication of application: **12.10.2011**
(21) Application number: 10461510.9
(22) Date of filing: 16.03.2010
(51) Int. Cl.: C07H 1/00, C07H 5/06

(54) **A process for the preparation of 1,6-anhydro-2-azido-3,4-di-O-benzyl-2-deoxy-D-glucopyranose**

(71) Applicant: Adamed sp. z o.o., 05-152 Czosnów k/Warszawy (PL)
(72) Inventor: Golinski, Jerzy, 03-352, Warszawa (PL); Kozlowski, Tomasz, 00-441, Warszawa (PL); Orkisz, Maria Jolanta, 01-930, Warszawa (PL)
(74) Representative: Sitkowska, Jadwiga

(57) **Abstract**

The invention relates to a process for the preparation of 1,6-anhydro-2-azido-3,4-di-O-benzyl-2-deoxy-D-glucopyranose, consisting of reaction of 1,6-anhydro-2-azido-2-deoxy-D-glucopyranose with benzyl bromide in a mixture of aromatic organic solvent and dipolar aprotic solvent and in the presence of a mixture of solid sodium hydroxide with solid potassium carbonate, phase transfer catalyst and tertiary alcohol.

1,6-anhydro-2-azido-3,4-di-O-benzyl-2-deoxy-D-glucopyranose is useful for preparation of 6-O-acetyl-2-azido-3,4-di-O-benzyl-2-deoxy-D-glucopyranose, a building block for synthesis of glucosamine oligosaccharides.

## Description

The invention relates to the process for the preparation of 1,6-anhydro-2-azido-3,4-di-O-benzyl-2-deoxy-D-glucopyranose, useful as an intermediate for the synthesis of oligosaccharides

1,6-anhydro-2-azido-3,4-di-O-benzyl-2-deoxy-D-glucopyranose is a known compound that finds its use in the carbohydrates chemistry, such as for the preparation of 6-O-acetyl-2-azido-3,4-di-O-benzyl-2-deoxy-D-glucopyranose. 6-0-acetyl-2-azido-3,4-di-O-benzyl-2-deoxy-D-glucopyranose is used as a precursor of active pharmaceutical ingredients, in particular oligosaccharides and sulphonated oligosaccharides containing D-glucosamine unit, such as for example sulphonated pentasaccharide fondaparinux.

The use of 6-O-acetyl-2-azido-3,4-di-O-benzyl-2-deoxy-D-glucopyranose of formula (I) as a glucosyl donor - monomeric building block for the synthesis of sulphonated oligosaccharides, including pentasaccharide fondaparinux, is described in the J.D.C. Codée et al J. Am. Chem. Soc. 2005, 127, 3767-3773. The process outlined there is presented in the scheme below.

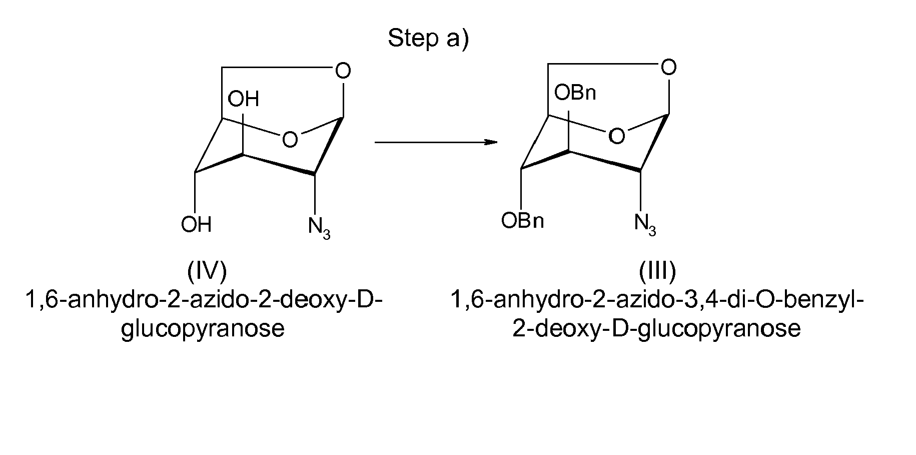

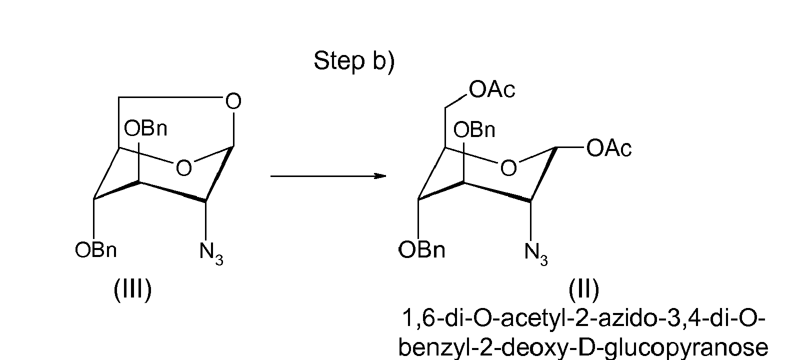

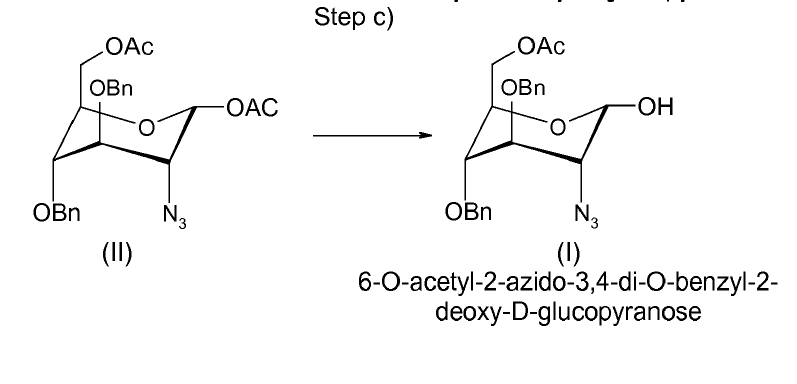

According to this scheme, 6-O-acetyl-2-azido-3,4-di-O-benzyl-2-deoxy-D-gluco-pyranose (I) is obtained from 1,6-anhydro-2-azido-2-deoxy-D-glucopyranose (IV) in three steps by a) benzylation with benzyl bromide in the presence of sodium hydride in DMF (with declared yield of 89%) in order to protect hydroxyl groups at positions 3 and 4, then in a second step b) acetylation in positions 1 and 6 with acetic anhydride in THF, and in the third step c) regioselective removal of the acetyl protection group from hydroxyl group in position 1 using 6% piperidine in THF. No further details of these three steps are given.

Benzylation of 1,6-anhydro-2-azido-3,4-di-O-benzyl-2-deoxy-D-glucopyranose with benzyl bromide in dimethylformamide in the presence of sodium hydride as an alkaline condensing agent is described in more detail in J. Hawley et al Eur.J.Org.Chem., 2002, 1925 - 1936. The disadvantages of the use of sodium hydride are its high price, inflammability and liberation of large amounts of inflammable hydrogen in contact with water and therefore necessity of keeping strictly anhydrous reaction medium.

Benzylation of 1,6-anhydro-2-azido-3,4-di-O-benzyl-2-deoxy-D-glucopyranose with benzyl bromide in dimethylformamide in the presence of barium hydroxide, with declared yield of 97%, is described in D.Tailler et al J.Chem.Soc. Perkin Trans 1, 1992, 3163-3164. The same reaction performed in dimethylformamide in the presence of mixture of barium oxide and barium hydroxide is described e.g. by F.Dasgupta, Per J.Garegg in Synthesis 1988, 626-628. These prior art processes using barium hydroxide are in turn inconvenient because of the necessity of removing from the reaction medium very toxic barium salts that form extremely fine precipitate requiring long filtration, as well as high prices of barium compounds. In addition, it appeared that in spite of long reaction times it is not possible to achieve high yields declared in the prior art.

Additionally, dimethylformamide used as a solvent in benzylation reaction in prior art processes is difficult to evaporate. The regeneration of dimethylformamide from aqueous solutions after work-up of reaction mixture is also difficult.

The known processes of benzylation with benzyl bromide conducted as in the above step a) proved useful only in a lab scale and scaling up was associated with technical problems.

W. Szeja and I. Fokt in Rec. Trav. Chim. Pays-Bas 108, 224-226 (1989) described benzylation of hydrophilic sugar polyols having from 3 to 8 hydroxyl groups with benzyl chloride using modified phase transfer catalysis method (PTC). The reaction is conducted in biphase liquid - liquid system 50% NaOH/organic phase or solid/liquid: NaOH+K₂CO₃/benzene, in the presence of quaternary ammonium salt as catalyst and tertiary alcohol as co-catalyst and/or co-solvent (e.g. DMSO), using a small excess of benzyl chloride.

It has been surprisingly found that this method can be used for benzylation of azide substituted sugar derivatives with benzyl bromide.

According to the invention, a process for the preparation of 1,6-anhydro-2-azido-3,4-di-O-benzyl-2-deoxy-D-glucopyranose is proposed, comprising benzylation of 1,6-anhydro-2-azido-2-deoxy-D-glucopyranose with benzyl bromide in an apolar organic solvent with the addition of dipolar aprotic solvent and in the presence of a mixture of solid sodium hydroxide with solid potassium carbonate, phase-transfer catalyst and tertiary alcohol.

Technique of carrying out phase-transfer catalyzed reactions is known as such. The reaction according to the process of invention is carried out with vigorous stirring. Conveniently cooling is applied to maintain temperature in the range preferably not exceeding 28-32°C.

According to the invention, an apolar organic solvent can be an aromatic solvent such as benzene or toluene, apolar chlorohydrocarbon solvent, such as methylene chloride, or ether solvent such as tetrahydrofuran or dioxan, and mixtures of these solvents. A preferred solvent for use in technical scale is toluene. Drying the toluene solution during the treatment of reaction mixture can be easily performed by azeotropic distillation.

Any typical phase-transfer catalyst can be used in the process of the invention, such as tertiary amines and tert-alkylammonium salts, especially tert-alkyl ammonium salts, such as chlorides, bromides, iodides and tert-alkylammonium bisulphates. Phase-transfer catalysts are well-known and described for example by W.E. Keller, in Phase-Transfer Reaction. Fluka-Compendium, vol. 1 i 2, Thieme Verlag, Stutgart 1979, and in C. M. Starks and C. Liotta, "Phase Transfer Catalysis, Principles and Techniques", Academic Press, N.Y., 1978.

Preferred phase transfer catalyst for the process according to the present invention is tert-butyl ammonium bromide.

In the process of the invention any known dipolar aprotic solvent can be used as dipolar aprotic solvent, and in particular can be selected from acetonitrile, dimethyl sulfoxide and dimethylformamide. Preferred dipolar aprotic solvent is dimethyl sulfoxide (DMSO). Dipolar aprotic solvent is used in a small amount relative to apolar organic solvent. Typically, the amount of dipolar aprotic solvent is in the range of 5 to 30% by weight in relation to aprotic dipolar solvent, preferably 5 to 20 % by weight, for example, about 15% by weight.

A tertiary alcohol can be in particular C1-C5 alcohol, especially tert-butyl alcohol or tert-amyl alcohol (2-methyl-2-butanol), most preferably tert-amyl alcohol.

Preferably, a mixture of sodium hydroxide and potassium carbonate in a weight ratio 1:4 is used. It is not necessary to keep anhydrous reaction medium, and therefore there is no need for dehydration of the solvents used.

In order to isolate the product, the reaction mixture is subjected to a standard work-up consisting of filtration of inorganic salts, washing the organic phase with water and subsequent concentration. The product is obtained after a relatively short reaction time with very good yield and purity. No additional purification is required before its use for subsequent synthesis. Alternatively, the product can be also purified by crystallization, e.g. from hexane.

### Example 1,6-Anhydro-2-azido-3,4-di-O-benzyl-2-deoxy-D-glucopyranose.

The reactor with a capacity of 20 liters is charged with 0.835 kg (4.47 mol) of 1,6-anhydro-2-azido-2-deoxy-D-glucopyranose, 0.715 kg (17.88 moles) of sodium hydroxide, 2.86 kg of anhydrous potassium carbonate, 14.05 kg of toluene, 1.97 kg of dimethyl sulfoxide, 0.179 kg of 2-methyl-2-butanol, 1.56 kg of benzyl bromide and 0.152 kg of tert-butylammonium bromide. The mixture is stirred for 4 hours, while cooling with water and maintaining temperature in the range of 28°-32°C. Then the mixture is filtered and inorganic salts are washed on a nutch filter with 2 x 2.65 kg of toluene. The combined toluene layer (filtrates) are washed in the reactor with 3 x 8.9 kg of water, dried with 0.45 kg of anhydrous sodium sulphate, filtered off, and washed with 1 kg of toluene. Combined toluene layers are evaporated under vacuum using 50°C water bath. The obtained 1.70 kg of residue after addition of 1.70 kg of n-hexane is heated to 55°C, then cooled to 25°C and transferred into the refrigerator (5°C) for 18 hours. The precipitate thus obtained is washed with 0.7 kg of cold n-hexane (+5°C) and dried on air to yield 1.569 kg of 1,6-anhydro-2-deoxy-3,4-di-O-benzyl-2-azido-D-glucopyranose with purity of 99.1% (HPLC). The practical yield of the synthesis is 95.7%
M.p.= 68°C-69°C.
¹H NMR, (Varian 500MHz), CDCl₃, TMS, δ= 3.27 (s, 1 H); 3.38 (s, 1 H); 3.65 (m, 1 H); 3.71 (dd, J=7.09Hz, J=5.98Hz, 1 H); 3.99 (dd, J=7.32Hz, J=0.88Hz, 1 H); 4.49-4.63 (m,5H); 5.49 (s,1 H); 7.28-7.37 (m,10H) ppm.

## Claims

1. A process for the preparation of 1,6-anhydro-2-azido-3,4-di-O-dibenzyl-2-deoxy-D-glucopyranose comprising reaction of 1,6-anhydro-2-azido-2-deoxy-D-glucopyranose with benzyl bromide **characterized in that** the reaction is performed in the mixture of aromatic organic solvent and dipolar aprotic solvent, in the presence of phase transfer catalyst and tertiary alcohol.

2. The process according to claim 1 **characterized in that** the aromatic solvent is toluene.

3. The process according to claim 1 or 2 **characterized in that** the phase transfer catalyst is tert-butylammonium bromide.

4. The process according to any of the claims 1 - 3 **characterized in that** the dipolar aprotic solvent is dimethylsulfoxide.

5. The process according to any of the claims 1 - 4 **characterized in that** the tertiary alcohol is tert-amyl alcohol.

6. The process according to any of the claims 1 - 5 **characterized in that** the weigh ratio of sodium hydroxide to potassium carbonate is 1:4.
